# EUROPÄISCHE PATENTSCHRIFT

(11) **EP 3 997 456 B1**
(45) Veröffentlichungstag und Bekanntmachung des Hinweises auf die Patenterteilung: **21.10.2026**
(21) Anmeldenummer: 20739334.9
(22) Anmeldetag: 08.07.2020
(51) Int. Cl.: G01N 31/22, A61B 90/70, A61B 90/96, A61B 90/98, A61L 2/28

(54) **ZUSAMMENSETZUNG ZUR HERSTELLUNG EINER PRÜFANSCHMUTZUNG ZUR BEWERTUNG DER REINIGUNGSWIRKUNG VON REINIGUNGSVORRICHTUNGEN UND DEREN VERWENDUNG**
COMPOSITION FOR PRODUCING TEST SOIL FOR EVALUATING THE CLEANING EFFECT OF CLEANING DEVICES AND USE OF SAME
COMPOSITION PERMETTANT DE PRODUIRE UNE SOUILLURE TEST POUR ÉVALUER L'ACTION DE NETTOYAGE DE DISPOSITIFS DE NETTOYAGE ET UTILISATION DE LADITE COMPOSITION

(30) Priorität: 09.07.2019 DE 102019210089
(43) Veröffentlichungstag der Anmeldung: 18.05.2022
(73) Patentinhaber: MEIKO Maschinenbau GmbH & Co. KG, 77652 Offenburg (DE)
(72) Erfinder: HOFER, Martin, 77770 Durbach, Ebersweier (DE); SIMUNDIC, Mario, 77797 Ohlsbach (DE)
(74) Vertreter: Altmann Stößel Dick Patentanwälte PartG mbB
(86) Internationale Anmeldenummer: PCT/EP2020/069257
(87) Internationale Veröffentlichungsnummer: WO 2021/005111

(56) Entgegenhaltungen:
- DE-A1- 19 602 673
- US-A1- 2002 168 622
- US-A1- 2014 099 233
- ANONYMOUS: "DIN ISO/TS 15883-5:2006-02 Reinigungs-Desinfektionsgeräte - Teil 5: Prüfanschmutzungen und -verfahren zum Nachweis der Reinigungswirkung (ISO/TS 15883-5:2005); Deutsche Fassung CEN ISO/TS 15883-5:2005", DIN ISO/TS, DE, February 2006 (2006-02-01), pages 1 - 82, XP009540428, DOI: 10.31030/9671530
- KELLY K E ET AL: "Chemically defined, clinically relevant test soils for cleaning validation of reusable medical devices", CENTRAL SERVICE, vol. 2015, no. 6, June 2015 (2015-06-01), pages 418 - 423, XP093334910
- A BLACKY ET AL: "Leitlinie für die Prüfung von Reinigungs-Desinfektionsgeräten mit thermischer Desinfektion für Steckbecken und Harnflaschen", 1 June 2017 (2017-06-01), pages 1 - 11, XP055746728, Retrieved from the Internet <URL:https://oegsv.com/wp/wp-content/uploads/10-OEGSV-LL-Pruefung-v.-RDG-S-2017-06.pdf> [retrieved on 20201103]
- S KRÜGER ET AL: "Testanschmutzungen (TA) für die Kontrolle von Dekontaminationsgeräten", 5 July 2003 (2003-07-05), pages 1 - 30, XP055747141, Retrieved from the Internet <URL:http://www.deconidi.ie/html/educ/lectures/efhss2003_0704_1205_Krueger_TestanschmutzungenWDs_de.pdf> [retrieved on 20201104]

## Beschreibung

### Technisches Gebiet

Die Erfindung betrifft eine Zusammensetzung, die die folgenden Bestandteile in trockener Form umfasst: (a) Nigrosin, (b) Weizenmehl und (c) Vollei. Die Zusammensetzung gemäß der Erfindung ist in Anspruch 1 definiert. Die Erfindung betrifft auch die Verwendung der erfindungsgemäßen Zusammensetzung zur Herstellung von Prüfanschmutzung zur Bewertung der Reinigungswirkung von Reinigungsvorrichtungen. Auch wird ein Verfahren bereitgestellt zur Herstellung einer Prüfanschmutzung zur Bewertung der Reinigungswirkung von Reinigungsvorrichtungen zur Reinigung von Behältern für menschliche Ausscheidungen, insbesondere von Reinigungs-Desinfektionsgeräten, umfassend den Schritt des Lösen der erfindungsgemäßen Zusammensetzung in einem wässrigen Medium. Die Erfindung betrifft eine Prüfanschmutzung, die die Bestandteile der erfindungsgemäßen Zusammensetzung in einem wässrigen Medium enthält, sowie deren Verwendung zur Bewertung der Reinigungswirkung von Reinigungsvorrichtungen. Schließlich betrifft die Erfindung ein Verfahren zur Bewertung der Reinigungswirkung von Reinigungsvorrichtungen zur Reinigung von Behältern für menschliche Ausscheidungen.

### Technischer Hintergrund

Aus dem Stand der Technik sind eine Vielzahl von Reinigungsvorrichtungen und Reinigungsverfahren zur Behandlung von Behältern zur Aufnahme menschlicher Ausscheidungen bekannt, wie beispielsweise Steckbecken oder Urinflaschen. Die zu behandelnden Behälter können größere Flüssigkeitsmengen oder Mengen an Feststoffabfällen enthalten, welche üblicherweise während der Reinigung entsorgt werden müssen. Zudem können derartige Behälter infektiöse Abfälle enthalten oder anderweitig kontaminiert sein, so dass, neben einer Entleerung, auch üblicherweise eine Desinfektion erforderlich ist. Derartige Reinigungsgeräte zur Behandlung von Behältern für menschliche Ausscheidungen werden dementsprechend häufig auch als Reinigungs- und Desinfektionsgeräte (RDG) oder auch als Reinigungs-Desinfektionsgeräte bezeichnet. Da in vielen Fällen unter anderem Steckbecken gereinigt werden, werden derartige Geräte häufig auch als Steckbeckenspüler bezeichnet. Anforderungen an derartige Reinigungsvorrichtungen werden grundsätzlich in der ISO-Normenreihe 15883 beschrieben.

Für mögliche Beispiele von Reinigungs- und Desinfektionsgeräten kann exemplarisch auf DE 10 2004 056 052 A1, DE 103 48 344 A1, EP 1 824 373 B1, EP 2 755 695 B1, DE 10 2013 203 342 A1 oder auf DE 10 2013 208 060A1 verwiesen werden. Die dort beschriebenen Reinigungs- und Desinfektionsgeräte sind exemplarisch auch im Rahmen der vorliegenden Erfindung einsetzbar.

Eine wesentliche Herausforderung für die Konstruktion und den Betrieb von Reinigungs- und Desinfektionsgeräten besteht in einer Sicherstellung der Reinigungswirkung und der Desinfektionswirkung. Hierzu existieren zahlreiche Normen, Vorgaben und Empfehlungen. Beispielsweise beschreibt DIN EN ISO 15883-6 allgemein die Anforderungen und Prüfverfahren für RDGs.

Neben der Sicherstellung der thermischen Desinfektionswirkung ist insbesondere eine ausreichende Befreiung von Verschmutzungen sicherzustellen. So empfiehlt beispielsweise die Deutsche Gesellschaft für Krankenhaushygiene die Verwendung von Prüfanschmutzungen, auch als Testanschmutzungen bezeichnet, um sichtbare Restverschmutzungen zu erkennen.

Aus der US 2014/0099233 A1 ist eine getrocknete Testanschmutzung für medizinische Instrumente bekannt, die im wesentlichen einen Analyten (z.B. Adenosintriphosphat) sowie ein polymeres Bindemittel enthält.

In der Vor-Norm CEN ISO/TS 15883-5 ist ausgeführt, dass als Nachweis der Reinigungswirksamkeit von RDGs nach der ISO-Normenreihe 15883 diejenigen Prüfanschmutzungen und Verfahren gültig sind, die gegenwärtig in einer Reihe von Ländern eingesetzt werden, und nationale Prüfanschmutzungen werden aufgelistet. So sind beispielsweise für Steckbecken und Urinflaschen in Deutschland Mischungen aus Rinderalbumin, Mucin, Maisstärke und E. faecium bekannt. In Österreich werden für Steckbecken Prüfanschmutzungen verwendet, welche Kartoffelflocken, Mehl, Nigrosin und Ei enthalten, so genannte KMNE-Prüfanschmutzungen, und für Urinflaschen Mischungen aus Mehl, Nigrosin und Ei, so genannte MNE-Prüfanschmutzungen. Weiterhin werden Vorgaben hinsichtlich der Herstellung der Prüfanschmutzungen sowie deren Lagerung bereitgestellt, wobei die Vorgaben auch Anweisungen zu den bei der Herstellung zu verwendenden Mengen der einzelnen Komponenten enthalten. Dabei werden durch Kochen zunächst getrennt Suspensionen aus Nigrosin und Weizenmehl hergestellt und gemischt. Unmittelbar vor Gebrauch werden Hühnereier hinzugegeben und die Mischung erwärmt. Weiterhin werden gegebenenfalls die Trockenkartoffelflocken hinzugegeben, bis die erforderliche Konsistenz erreicht ist. Mit derartigen Prüfanschmutzungen werden dann Prüfkörper in Form von Steckbecken oder Urinflaschen mit vorgegebenen Mengen befüllt und die Prüfanschmutzung in den Prüfkörpern verteilt. Anschließend werden die RDGs mit den angeschmutzten Prüfkörpern voll beladen, und es wird ein entsprechender Reinigungszyklus durchgeführt. Anschließend wird eine Sichtprüfung vorgenommen, wobei das Akzeptanzkriterium darin besteht, dass keine Rückstände der Prüfanschmutzung am Prüfkörper mehr sichtbar sein dürfen.

Eine Herausforderung in der Praxis besteht darin, dass derartige Prüfanschmutzungen in ihrer Herstellung aufwändig sind. Die Prüfanschmutzungen können im Kühlschrank maximal drei Tage aufbewahrt werden und sollten grundsätzlich unmittelbar nach ihrer Herstellung verwendet werden. Wartungsarbeiten und Kontrollen sind jedoch in der Regel am Aufstellungsort der Reinigungsvorrichtungen vorzunehmen. Gerade dort stehen jedoch häufig erforderliche Vorrichtungen zur Herstellung der Prüfanschmutzungen nicht zur Verfügung. Weiterhin ist die Herstellung derartiger Prüfanschmutzungen zeitaufwändig und stellt häufig einen maßgeblichen Schritt für die Dauer der Prüfung dar.

### Aufgabe der Erfindung

Es wäre daher wünschenswert, Prüfanschmutzungen und Verfahren zur Bewertung der Reinigungswirkung von Reinigungsvorrichtungen für menschliche Ausscheidungen bereitzustellen, welche die Nachteile bekannter Prüfanschmutzungen und Verfahren der genannten Art weitgehend vermeiden. Insbesondere soll der Aufwand für die Bereitstellung der Prüfanschmutzungen sowie der Aufwand für die Durchführung des gesamten Verfahrens verringert werden.

### Beschreibung der Erfindung

Diese Aufgabe wird durch die Ausführungsformen gemäß den unabhängigen Patentansprüchen adressiert. Vorteilhafte Weiterbildungen sind in den abhängigen Ansprüchen dargestellt.

Im Folgenden werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne Einschränkung der Möglichkeit, dass das Merkmal oder Element einfach oder mehrfach vorgesehen sein kann.

Weiterhin werden im Folgenden die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten, die hierdurch eingeleiteten Merkmale mit anderen Merkmalen zu kombinieren, seien es optionale oder nicht-optionale Merkmale, unangetastet bleiben.

Die Erfindung betrifft eine Zusammensetzung, die die folgenden Bestandteile in trockener Form umfasst: (a) Nigrosin, (b) Weizenmehl und (c) Vollei; die Zusammensetzung gemäß der vorliegenden Erfindung ist in Anspruch 1 definiert.

Der Begriff "Zusammensetzung" beschreibt eine Mischung von mehreren Bestandteilen. Die Bestandteile können dabei in unterschiedlichen Anteilen vorliegen. Dabei liegen die Bestandteile in der erfindungsgemäßen Zusammensetzung in trockener Form vor. Die Bestandteile können in jeder beliebigen trockenen Form vorliegen. Abhängig vom Bestandteil kann dieser in kristalliner oder amorpher Form vorliegen. Die Bestandteile können als kristallines oder amorphes Pulver in der Zusammensetzung vorliegen oder in anderer geeigneter Form, z.B. als Puder, granulares Material oder Granulat. Typischerweise ist die Zusammensetzung gemäß der vorliegenden Erfindung ein rieselfähiger Feststoff.

Unter dem Begriff "trockene Form" ist erfindungsgemäß zu verstehen, dass die Bestandteile, die von der erfindungsgemäßen Zusammensetzung umfasst sein sollen, im Wesentlichen frei von Flüssigkeiten, insbesondere von wässrigen Lösungsmitteln, sein sollen. Unter "im Wesentlichen frei von Flüssigkeiten" ist hierbei zu verstehen, dass die Restflüssigkeit in der in der erfindungsgemäß bereitgestellten Zusammensetzung nicht genügt, um Bestandteile zu lösen, so dass die Bestandteile in der Zusammensetzung selbst auch als Feststoffe vorliegen.

Abhängig vom Bestandteil kann sich der Gehalt an Restflüssigkeit unterscheiden. Bei Weizenmehl beträgt der Restflüssigkeitsgehalt bevorzugt höchstens 20 Gew.-%, höchstens 15 Gew.-%, höchstens 10 Gew.-%, höchstens 5 Gew.-% oder besonders bevorzugt höchstens 2 Gew.-% bezogen auf das Gesamtgewicht des in der Zusammensetzung verwendeten Bestandteils. Im Volleipulver beträgt der Restflüssigkeitsgehalt vorzugsweise höchstens 10 Gew.-%, höchstens 7 Gew.-%, höchstens 5 Gew.-%, oder besonders bevorzugt höchstens 2 Gew.-% bezogen auf das Gesamtgewicht des in der Zusammensetzung verwendeten Bestandteils. Nigrosin Pulver ist vorzugsweise frei von nachweisbarer eingelagerter Restflüssigkeit. Sofern Wasser eingelagert ist, handelt es sich um gebundenes Kristallwasser.

Der Begriff "Nigrosin" wie hier verwendet bezeichnet einen blauschwarzen, anionischen Farbstoff aus der Gruppe der Diaminophenazin-Verbindungen, der wasserlöslich ist. Neben dem wasserlöslichen Nigrosin gibt es auch noch Nigrosine, die alkohol- oder öllöslich sind. Bevorzugt im Sinne der Erfindung ist ein wasserlösliches Nigrosin mit CAS Nummer 8005-03-6. Nigrosine sind kommerziell erhältlich. Nigrosine können durch Erhitzen von Nitrobenzol, Anilin und Anilinhydrochlorid mit metallischem Eisen und Eisen(III)-chlorid gewonnen werden. Besonders bevorzugte Nigrosine sind in den Ausführungsbeispielen näher beschrieben.

Der Begriff "Weizenmehl" ist ein Pulver, das beim Mahlen von Weizengetreidekörnern entsteht. Von dem Begriff, wie hierin verwendet, umfasst sind Mehle von allen Weizenarten, einschließlich der Unterarten Dinkel, Emmer oder Einkorn. Weizenmehle werden abhängig von ihren Backeigenschaften, ihrem Mahlgrad, das heißt der Partikelgröße, ihrem Aschegehalt und ihrem Proteingehalt typisiert. Bevorzugt kann das Weizenmehl glattes, griffiges oder doppelgriffiges Weizenmehl sein. Bevorzugt ist das erfindungsgemäß eingesetzte Mehl ein Weizenmehl Typ 405. Andere Typen, die ebenfalls verwendet werden können, sind Typ 550, 812, 1050 oder 1600. Besonders bevorzugtes Weizenmehl ist in den Ausführungsbeispielen näher beschrieben.

Der Begriff "Vollei", wie hier verwendet, bezeichnet ein Präparat, das im Wesentlichen die Bestandteile von Eiern (Dotter und Eiklar) aufweist. Insbesondere soll das Präparat die Proteine, Lipide und Kohlehydrate aus Eiern umfassen. Die Eier sind erfindungsgemäß bevorzugt Hühnereier, wobei aber auch andere Eier eingesetzt werden können, insbesondere Geflügeleier, z.B. Wachteleier oder Straußeneier. Erfindungsgemäß liegen die Bestandteile des Volleis ebenfalls in trockener Form vor. Hierzu kann Vollei durch geeignete Verfahren getrocknet werden. Hierzu zählen z.B. Lyophilisations-Verfahren. Erfindungsgemäß vom Begriff umfasst sind auch Vollei-Substitute, z.B. vegetarisches oder veganes Vollei oder artifizielle Zusammensetzungen, die die Bestandteile von Vollei umfassen. Besonders bevorzugt handelt es sich bei dem Vollei im Sinne der Erfindung um Hühnerei-Pulver. Besonders bevorzugtes Vollei ist in den Ausführungsbeispielen näher beschrieben.

Die erfindungsgemäße Zusammensetzung kann in wässrigen Lösungsmitteln wie Wasser oder wässrigen Pufferlösungen gelöst werden. Hierzu wird die Zusammensetzung in einer geeigneten Menge des wässrigen Lösungsmittels gelöst. Der Lösungsvorgang kann hierbei durch geeignete Maßnahmen, z.B. automatisches oder manuelles Rühren, unterstützt werden. Die gelöste Zusammensetzung kann dann als Prüfanschmutzung eingesetzt werden, um die Reinigungsleistung von Reinigungsvorrichtungen zur Reinigung von Behältern für menschliche Ausscheidungen zu bewerten. Abhängig von der Zusammensetzung kann die Reinigungsleistung für Steckbecken oder Urinflaschen bewertet werden. Geeignete Verfahren sind ebenfalls hierin an anderer Stelle beschrieben.

Die erfindungsgemäße Zusammensetzung kann als vorgefertigtes Gemisch der Bestandteile bereitgestellt werden. Hierzu können die Bestandteile der Zusammensetzung in geeigneter, trockener, Form, z.B. als Pulver, miteinander vermischt werden, so dass bevorzugt eine homogene Mischung der Bestandteile entsteht. Diese kann dann in geeigneten Verpackungen bereitgestellt werden. Die Verpackungen sollten hierbei verhindern, dass die Zusammensetzung feucht wird und somit ungewollte Lösung von Bestandteilen auftritt. Alternativ können die Bestandteile der erfindungsgemäßen Zusammensetzung auch einzeln in geeigneten Verpackungen bereitgestellt werden, z.B. als so genanntes Kit. In diesem Fall wird die Zusammensetzung vor oder bei der Herstellung der Prüfanschmutzung aus den individuellen Bestandteilen bereitgestellt. Typischerweise werden die Bestandteile hierbei in vordefinierten Mengen bereitgestellt, so dass die Bereitstellung der Zusammensetzung aus den Bestandteilen kein Abmessen der Bestandteile benötigt, sondern durch einfaches Mischen der verpackten individuellen Bestandteile in trockenem Zustand oder durch direktes Lösen in einem wässrigen Lösungsmittel bewerkstelligt werden kann.

Vorteilhafterweise kann die erfindungsgemäße Zusammensetzung, die ja aus trockenen Bestandteilen besteht, über lange Zeit gelagert werden. Derzeit verwendete Prüfanschmutzungen umfassen flüssiges Vollei und sind dementsprechend als wässrige Zusammensetzungen nur begrenzt haltbar. Die erfindungsgemäße Zusammensetzung erlaubt zudem die Bereitstellung einer standardisierten Prüfanschmutzung, da die Bestandteile der Zusammensetzung gut in einer definierten Menge eines wässrigen Lösungsmittels gelöst werden können. Schwankungen oder Probleme beim Lösen, wie sie bei der Verwendung von flüssigem Vollei auftreten können, können so weitestgehend vermieden werden.

Die erfindungsgemäße Zusammensetzung kann vorzugsweise in Form von portionierten Einheiten bereitgestellt werden. Unter einer "portionierten Einheit" im Sinne der vorliegenden Erfindung kann vorzugsweise eine bestimmte Menge der Zusammensetzung verstanden werden. Besonders bevorzugt beinhaltet eine "portionierte Einheit" die benötigte Menge der Zusammensetzung bezogen auf ein zuzusetzendes Volumen von 250 ml wässriges Lösungsmittel. Ganz besonders bevorzugt enthält eine portionierte Einheit eine Menge der Zusammensetzung von 47,8 g bis 62,2 g und am meisten bevorzugt 55 g.

Portionierte Einheiten können vorteilhaft sein, da der Anwender die notwendige Menge der Testanschmutzung nicht abwiegen muss. Somit kann die Handhabung und der Gebrauch der Testanschmutzung zusätzlich vereinfacht werden.

Die portionierten Einheiten können in geeigneter Weise verpackt sein. Besonders geeignete Formen der Verpackung sind Portionsbeutel, oder andere Formen der Verpackung portionierter Einheiten, wie Tüten, Sachets, Schläuche, Becher, Kartons. Entsprechend kann die erfindungsgemäße Zusammensetzung insbesondere in Form einer portionierten Einheit, vorzugsweise in einem Portionsbeutel vorliegen.

Die oben genannten geeigneten Formen der Verpackung können mit einer Kennzeichnung, beispielsweise einem Aufdruck, Etikett, Aufkleber oder Label versehen sein, die einen grafischen Code und/oder einen RFID- Tag umfasst. Bei dem grafischen Code kann es sich beispielsweise um einen eindimensionalen Strichcode (Barcode) oder um einen zweidimensionalen Code (QR- Code) handeln. Dieser Code kann beim Auslesen, beispielsweise mit einem Smartphone oder einem anderen mobilen Endgerät, zu einer Ablage im Internet, wie einer Website, leiten, auf der für den Benutzer eine Anleitung zur Verwendung der Testanschmutzung und Durchführung des Tests angezeigt werden kann. Auch der RFID-Tag kann beim Auslesen, beispielsweise mit einem Smartphone oder einem anderen mobilen Endgerät, den Anwender anleiten wie die Testanschmutzung anzuwenden ist und/oder wie der Test durchgeführt werden soll. Hierdurch kann die Handhabung und der Gebrauch der Testanschmutzung zusätzlich vereinfacht werden.

Nachfolgend werden bevorzugte Ausführungsformen der erfindungsgemäßen Zusammensetzung beschrieben.

Bevorzugt liegen Nigrosin und Vollei als Pulver vor. Gemäß der Erfindung liegen die Bestandteile in folgenden Mengen jeweils bezogen auf ein zuzusetzendes Volumen von 250 ml wässriges Lösungsmittel in der Zusammensetzung vor: (a) Nigrosin 0,8 g bis 1,2 g, vorzugsweise 1,0 g, (b) Weizenmehl 30 g bis 40 g, vorzugsweise 35 g und (c) Vollei 17 g bis 21 g, vorzugsweise 19 g. Die Zusammensetzung hat somit ein Gesamtgewicht von 47,8 g bis 62,2 g und bevorzugt 55 g. In einer bevorzugten Zusammensetzung beträgt der Anteil an Nigrosin also etwa 1,8 Gew-%, der Anteil an Weizenmehl etwa 63,6 Gew-% und der Anteil an Vollei 34,6 Gew-%. Die Zusammensetzung gemäß der vorliegenden Erfindung kann entsprechend für beliebige Volumina an wässrigem Lösungsmittel vorbereitet werden. s. oben, jetzt in der Definition von Weizenmehl.

Bevorzugt umfasst die Zusammensetzung Kartoffeltrockenflocken als zusätzlichen Bestandteil. Typischerweise liegen die Kartoffeltrockenflocken in einer Menge von 35 g bis 45 g, vorzugsweise 40 g, bezogen auf ein zuzusetzendes Volumen von 250 ml wässrigen Lösungsmittels vor. Diese erfindungsgemäße Zusammensetzung hat dann somit ein Gesamtgewicht von 82,8 g bis 107,2 g und bevorzugt 95 g. In einer bevorzugten Zusammensetzung beträgt der Anteil an Nigrosin also etwa 1,0 Gew-%, der Anteil an Weizenmehl etwa 36,9 Gew-%, der Anteil an Vollei 20 Gew-% und der Anteil an Kartoffeltrockenflocken 42,1 Gew%.

Der Begriff "Kartoffeltrockenflocken", wie hier verwendet, bezeichnet Aggregate aus trockenen Kartoffelzellen. Kartoffeltrockenflocken sind kommerziell erhältlich und werden durch im Stand der Technik bekannte Verfahren hergestellt. Typischerweise werden hierzu Kartoffeln gekocht und zu Brei verarbeitet. Dieser Brei wird als dünne Schicht auf Walzentrocknern getrocknet und zu Flocken gewünschter Größe zerkleinert. Verfahrenstechnisch kann ein unterschiedlicher Anteil der Kartoffelzellen zerstört werden. Dadurch kann auch der Stärkegehalt unterschiedlich eingestellt werden. Besonders bevorzugte Kartoffeltrockenflocken sind in den Ausführungsbeispielen näher beschrieben. Von dem Begriff sind aber auch Kartoffelpulver umfasst.

Die Erfindung betrifft auch die Verwendung einer Zusammensetzung wie zuvor beschrieben, zur Herstellung von Prüfanschmutzung zur Bewertung der Reinigungswirkung von Reinigungsvorrichtungen zur Reinigung von Urinflaschen für menschliche Ausscheidungen.

Es versteht sich hierbei, dass die Prüfanschmutzung durch Lösen der erfindungsgemäßen Zusammensetzung in einem wässrigen Lösungsmittel zunächst bereitgestellt werden muss. Dies kann mit den hierin beschriebenen Verfahren geschehen. Bevorzugt wird die Prüfanschmutzung durch Lösen der Zusammensetzung in einem wässrigen Medium hergestellt, besonders bevorzugt ist das wässrige Lösungsmittel hierbei Wasser oder eine wässrige Pufferlösung. Die Prüfanschmutzung wird anschließend verwendet, um die Reinigungswirkung von Reinigungsvorrichtungen zu bewerten. Hierzu wird die Prüfanschmutzung auf die Urinflasche aufgetragen. Dies kann homogen oder punktuell auf einer inneren und/oder äußeren Oberfläche der Urinflasche erfolgen. Verfahren zum Auftragen der Prüfanschmutzung sind dem Fachmann bekannt.

Der Begriff "Reinigungsvorrichtung", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung beziehen, welche eingerichtet ist, um Reinigungsgut von Verunreinigungen zu befreien. Die Reinigungsvorrichtung kann insbesondere mindestens eine Reinigungskammer zur Aufnahme des Reinigungsguts umfassen, sowie eine Beaufschlagungsvorrichtung, mit welcher das Reinigungsgut mit mindestens einem Reinigungsfluid, beispielsweise mindestens einer Reinigungsflüssigkeit und/oder Dampf, beaufschlagt wird. Für Beispiele möglicher Reinigungsvorrichtungen kann auf den oben genannten Stand der Technik verwiesen werden.

Als Reinigungsgut im Sinne der Erfindung kommen alle Gegenstände in Betracht, wie sie üblicherweise vom Fachmann verstanden werden. Der Begriff ist nicht beschränkt auf eine spezielle oder angepasste Bedeutung. Der Begriff kann, ohne Beschränkung, sich insbesondere auf Behälter beziehen, welche beispielsweise im Krankenhaus- oder Pflegebereich eingesetzt werden können und welche beispielsweise feste oder flüssige Ausscheidungen in einer Menge von mindestens 100 ml, insbesondere in einer Menge von 200 ml oder sogar 500 ml, aufnehmen können. Insbesondere kann es sich bei diesen Behältern um Steckbecken und/oder Urinflaschen handeln.

Dementsprechend kann die Reinigungsvorrichtung insbesondere als Reinigungs-Desinfektionsgerät im Sinne beispielsweise der ISO-Normenreihe 15883 Reinigungs - und Desinfektionsgeräte, Teil 1: Allgemeine Anforderungen, Begriffe und Prüfverfahren ausgestaltet sein.

Der Begriff "Prüfanschmutzung", wie er hier verwendet wird, ist ein weiter Begriff, dem seine gewöhnliche und gängige Bedeutung beigemessen werden soll, wie der Fachmann sie versteht. Die Prüfanschmutzung im Sinne der Erfindung umfasst die erfindungsgemäße Zusammensetzung in gelöster Form wie hierin beschrieben. Die Prüfanschmutzung ist geeignet, einen Prüfkörper zu benetzen. Weiterhin ist die Entfernung der Prüfanschmutzung von dem Prüfkörper durch ein zu bewertendes Reinigungsverfahren oder durch eine zu bewertende Reinigungsvorrichtung nachweisbar. Die Prüfanschmutzung kann insbesondere als pastöse Masse ausgestaltet sein, welche mit dem menschlichen Auge erkennbar ist.

Der Begriff "Prüfkörper", wie er hier verwendet wird, ist ein weiter Begriff, dem seine gewöhnliche und gängige Bedeutung beigemessen werden soll, wie der Fachmann sie versteht. Der Begriff ist nicht beschränkt auf eine spezielle oder angepasste Bedeutung. Der Begriff kann, ohne Beschränkung, sich insbesondere auf eine Vorrichtung oder ein Element beziehen, welche einem Reinigungsverfahren unterzogen werden können und welche mindestens eine während des Reinigungsverfahrens gereinigte Oberfläche aufweisen, auf welche vor der Reinigung eine Prüfanschmutzung aufgebracht werden kann. Insbesondere kann es sich bei dem Prüfkörper um einen Behälter zur Aufnahme menschlicher Ausscheidungen handeln, insbesondere um ein Steckbecken und/oder um eine Urinflasche. Dementsprechend kann es sich bei dem Prüfkörper insbesondere um einen Prüfkörper im Sinne der Vor-Norm DIN ISO/TS 15883-5, Reinigungs-Desinfektionsgeräte - Teil 5: Prüfanschmutzungen und -verfahren zum Nachweis der Reinigungswirkung handeln.

Weiterhin betrifft die Erfindung die Verwendung einer Zusammensetzung wie zuvor beschrieben, sowie Kartoffeltrockenflocken als zusätzlichen Bestandteil, zur Herstellung von Prüfanschmutzung zur Bewertung der Reinigungswirkung von Reinigungsvorrichtungen zur Reinigung von Behältern für menschliche Ausscheidungen.

Die Erfindung betrifft auch ein Verfahren zur Herstellung einer Prüfanschmutzung zur Bewertung der Reinigungswirkung von Reinigungsvorrichtungen zur Reinigung von Behältern für menschliche Ausscheidungen, insbesondere von Reinigungs-Desinfektionsgeräten, umfassend den Schritt des Lösens der erfindungsgemäßen Zusammensetzung in einem wässrigen Medium.

Bevorzugt ist das wässrige Medium Wasser oder eine wässrige Pufferlösung. Die erfindungsgemäße Zusammensetzung wird in das wässrige Medium eingebracht und in diesem gelöst. Der Lösungsvorgang kann hierbei durch physikalische Methoden unterstützt werden. Hierzu kann das Lösen der Bestandteile der erfindungsgemäßen Zusammensetzung durch manuelles oder automatisches Rühren durchgeführt werden. Sowohl manuelle Rührer als auch automatische Rührgeräte, die für den Lösungsvorgang im Sinne der Erfindung geeignet sind, sind dem Fachmann bekannt. Das Lösen kann zudem durch Erwärmen der Lösung während des Lösens oder durch die Verwendung von erwärmtem wässrigen Medium als Lösungsmittel begünstigt werden. Typischerweise wird die Lösung oder das Lösungsmittel hierbei auf eine Temperatur im Bereich von 20°C bis 40°C, vorzugsweise unter 35°C, besonders vorzugsweise 25°C, 28°C, 30°C oder 35°C, erwärmt.

Die Erfindung betrifft weiterhin eine Prüfanschmutzung zur Bewertung der Reinigungswirkung von Reinigungsvorrichtungen zur Reinigung von Behältern für menschliche Ausscheidungen, insbesondere von Reinigungs-Desinfektionsgeräten, die die Bestandteile der erfindungsgemäßen Zusammensetzung in einem wässrigen Medium, vorzugsweise Wasser, umfasst oder die durch das erfindungsgemäße Verfahren erhältlich ist.

Die Erfindung betrifft die Verwendung der erfindungsgemäßen Prüfanschmutzung zur Bewertung der Reinigungswirkung von Reinigungsvorrichtungen zur Reinigung von Behältern für menschliche Ausscheidungen.

Die Prüfanschmutzung kann die Bestandteile (a) Nigrosin, (b) Weizenmehl und (c) Vollei der erfindungsgemäßen Zusammensetzung in einem wässrigen Medium, vorzugsweise Wasser, umfassen. Durch diese Prüfanschmutzung wird die Reinigungswirkung von Reinigungsvorrichtungen zur Reinigung von Urinflaschen bevorzugt bewertet.

Die Prüfanschmutzung kann die Bestandteile (a) Nigrosin, (b) Weizenmehl und (c) Vollei der erfindungsgemäßen Zusammensetzung und zusätzlich Kartoffeltrockenflocken in einem wässrigen Medium, vorzugsweise Wasser, umfassen. Durch diese Prüfanschmutzung wird die Reinigungswirkung von Reinigungsvorrichtungen zur Reinigung von Steckbecken bevorzugt bewertet.

Die Erfindung betrifft schließlich auch ein Verfahren zur Bewertung der Reinigungswirkung von Reinigungsvorrichtungen zur Reinigung von Behältern für menschliche Ausscheidungen, insbesondere von Reinigungs-Desinfektionsgeräten, umfassend:
(a) Herstellen von Prüfanschmutzung mit einem erfindungsgemäßen Verfahren wie zuvor beschrieben;
(b) Auftragen der in Schritt (a) hergestellten Prüfanschmutzung an Behältern für menschliche Ausscheidungen, insbesondere Steckbecken oder Urinflaschen;
(c) Reinigen der Behälter aus Schritt (c) mittels einer zu bewertenden Reinigungsvorrichtung; und
(d) Bewertung der Reinigungswirkung der Reinigungsvorrichtung zur Reinigung von Behältern für menschliche Ausscheidungen anhand der Menge der vorhandenen oder entfernten Prüfanschmutzung.

Das erfindungsgemäße Verfahren zur Bewertung der Reinigungswirkung von Reinigungsvorrichtungen kann hierbei bevorzugt eingesetzt werden, um die Reinigungswirkung für Steckbecken und/oder Urinflaschen bezogen auf menschliche Ausscheidungen zu bewerten. Abhängig von der Art der Ausscheidung bzw. der Art des Reinigungsgutes (Steckbecken oder Urinflasche) kann eine entsprechend geeignete erfindungsgemäße Zusammensetzung ausgewählt werden. Für Urinflaschen wird bevorzugt eine Zusammensetzung ohne und für Steckbecken mit Kartoffeltrockenflocken verwendet.

Mit der entsprechend gewählten erfindungsgemäßen Zusammensetzung wird zunächst eine Prüfanschmutzung durch Lösen der Zusammensetzung in einem wässrigen Medium wie hierin beschrieben hergestellt.

Diese Prüfanschmutzung wird auf die jeweiligen Prüfkörper aufgetragen und angetrocknet. Hierbei kann der Auftrag an einer oder mehreren inneren und/oder äußeren Oberflächen homogen oder punktuell erfolgen. Die Prüfanschmutzung wird bevorzugt für etwa 5 bis 10 min, besonders bevorzugt 5 bis 7 min angetrocknet. Typischerweise geht die Prüfanschmutzung in diesem Zustand eine durch den Reinigungsvorgang reversible, aber feste Verbindung mit der Oberfläche des Prüfkörpers ein.

Die so hergestellten Prüfkörper werden in der zu bewertenden Reinigungsvorrichtung mit einem geeigneten Reinigungsprogramm gereinigt.

Die gereinigten Prüfkörper werden anschließend hinsichtlich der Reinigungsleistung der Reinigungsvorrichtung oder des verwendeten Reinigungsprogramms bewertet. Hierzu wird bevorzugt die Menge der vorhandenen oder entfernten Prüfanschmutzung auf den Prüfkörpern bestimmt und mit einer Referenz verglichen. Als Referenz kann ein Prüfkörper ohne Prüfanschmutzung oder ein ungereinigter Prüfkörper mit Prüfanschmutzung dienen. Alternativ kann die Referenz auch ein für den Prüfkörper definierter Zustand sein. Der Vergleich kann hierbei durch Inaugenscheinnahme oder durch Vergleich, bevorzugt automatisierten Vergleich, von gemessenen Parametern erfolgen, die Indikatoren für die An- oder Abwesenheit der Prüfanschmutzung sind, erfolgen. Geeignete Parameter können hierbei Parameter aus der Auswertung von Bilddaten oder optischen Messungen sein. Entsprechende Verfahren sind dem Fachmann wohlbekannt.

Anhand der Menge der entfernten Prüfanschmutzung kann die Reinigungsleistung bewertet werden. Die entfernte Menge kann hierbei als Bewertungsindikator für die Reinigungsleistung dienen, wobei eine große entfernte Menge an Prüfanschmutzung ein Indikator für eine gute, eine geringe Menge an entfernter Prüfanschmutzung ein Indikator für schlechte Reinigungsleistung ist. Hierzu kann auch ein Scoringsystem verwendet werden, wonach die Reinigungsleistung in Abhängigkeit der entfernten Menge an Prüfanschmutzung in verschiedenen Kategorien eingeteilt wird.

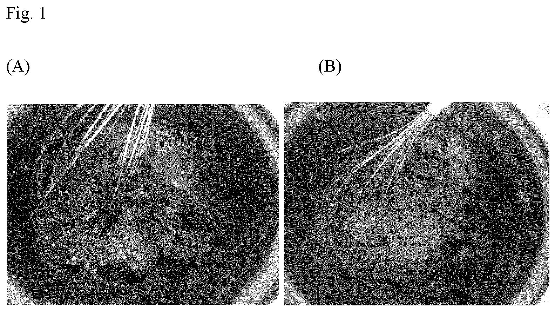

**Figur** 1 zeigt einen optischen Vergleich der Prüfanschmutzungen nach KMNE (Koller) (A) und gemäß der vorliegenden Erfindung (B).

### Beschreibung der Ausführungsbeispiele

Die nachfolgenden Ausführungsbeispiele sollen die Erfindung illustrieren. Sie dienen auf keinen Fall dazu, den Schutzbereich zu beschränken.

### Beispiel 1: Prüfanschmutzung für Steckbecken

Prüfanschmutzung für das Steckbecken: 1,0 gr. Nigrosinpulver (C.I.50420 wasserlöslich für die Mikroskopie) 35 gr. Weizenmehl Typ 405 40 gr. Kartoffeltrockenflocken (Kartoffelpüree) 19 gr. Trocken-Volleipulver (sprühgetrocknet): Gut vermischen, kann luftdicht verpackt ca.12 Monate aufbewahrt und verwendet werden. Vor Gebrauch die Mischung in einen Topf geben und mit 250 ml lauwarmem Wasser mit einem Schneebesen 2-3 min anrühren. Um die Konsistenz der Masse zu prüfen, werden die Schlaufen des Schneebesens zunächst vollständig in die Masse eingetaucht und anschließend wieder aus der Masse herausgezogen. Wenn die Mischung die richtige Konsistenz besitzt, fließt sie langsam an den Schlaufen des Schneebesens herunter, und nach 5-10 Sekunden weist der im Schneebesen verbleibende Mischungsklumpen einen Durchmesser von 40- 50 mm auf. Reicht als Anschmutzung für 1-2 Steckbecken oder Stuhleimer. Masse nach Vorschrift auf die Steckbecken bzw. Stuhleimer auftragen. Nach dem Auftragen die Masse 5-7 min antrocknen lassen. Innerhalb von 3-4 Stunden sollte diese zubereitete Masse verwendet werden.

### Beispiel 2: Prüfanschmutzung für Urinflaschen

Prüfanschmutzung für die Urinflasche: 1,0 gr. Nigrosinpulver (C.I.50420 wasserlöslich für die Mikroskopie) 35 gr. Weizenmehl Typ 405 19 gr. Trocken-Volleipulver: Gut vermischen, kann luftdicht verpackt ca.12 Monate aufbewahrt und verwendet werden. Vor Gebrauch die Mischung in einen Topf geben und mit 250 ml lauwarmem Wasser mit einem Schneebesen 2-3 min anrühren. Masse nach Vorschrift in/auf die Urinflasche auftragen. Nach dem Auftragen die Masse 5-7 min antrocknen lassen. Innerhalb von 3-4 Stunden sollte diese zubereitete Masse verwendet werden

### Beispiel 3: Prüfung der Reinigungswirkung

Prüfanschmutzungen werden wie in den Beispielen 1 und 2 beschrieben hergestellt und auf Steckbecken oder Urinflaschen aufgetragen und angetrocknet. Die so bereitgestellten Prüfkörper werden einem Reinigungs- und Desinfektionsverfahren in einer Reinigungsvorrichtung in geeigneter Weise unterzogen. Anhand der verbleibenden Prüfanschmutzung auf den Prüfkörpern (Vorher-Nachher-Vergleich) kann die Reinigungsleistung bewertet werden.

### Vergleichsbeispiel

: Vergleich der Prüfanschmutzung für Steckbecken nach Koller mit Zubereitung für den KMNE-Schmutz zur Prüfung von Reinigung und Desinfektion (Koller):
1. Nigrosinlösung: 6 g Nigrosinpulver in 276ml handwarmes Wasser einrühren.
2. Mehlkleister: 53 g doppelgriffiges Weizenmehl in 369 ml kaltes Wasser einrühren und etwa drei Minuten lang einkochen.
3. Die Lösung und den Kleister gut vermischen und auf Zimmertemperatur (ca.35°) abkühlen lassen.
4. Drei verquirlte frische Eier unter die Masse mischen und anschließend 100 g Kartoffelteigflocken in kleinen Portionen unterrühren.

Die Endkonsistenz als Stuhlersatz: Bei dem Eintauchen einer Schneerute - von einer Tiefe von 7cm - sollte ein Klumpen von 4 bis 5cm Durchmesser für 5 bis 10 Sekunden hängen bleiben.

Diese Masse wurde mit einer Masse, die wie in Beispiel 1 beschrieben hergestellt wurde, verglichen. Die Ergebnisse sind in Figur 1 gezeigt. Es besteht hinsichtlich der Prüfungsrelevanten Eigenschaften kein wesentlicher Unterschied.

## Patentansprüche

1. Zusammensetzung, die die folgenden Bestandteile in trockener Form umfasst: (a) Nigrosin, (b) Weizenmehl und (c) Vollei oder Vollei-Substitute, wie vegetarisches oder veganes Vollei oder artifizielle Zusammensetzungen, die die Bestandteile von Vollei umfassen, wobei die Bestandteile in folgenden Mengen jeweils bezogen auf ein zuzusetzendes Volumen von 250 ml wässriges Lösungsmittel in der Zusammensetzung vorliegen: (a) Nigrosin 0,8 g bis 1,2 g, vorzugsweise 1,0 g, (b) Weizenmehl 30 g bis 40 g, vorzugsweise 35 g und (c) Vollei 17 g bis 21 g, vorzugsweise 19 g.

2. Zusammensetzung nach Anspruch 1, wobei Nigrosin und Vollei als Pulver vorliegen.

3. Zusammensetzung nach einem der Ansprüche 1 oder 2, wobei die Zusammensetzung Kartoffeltrockenflocken als zusätzlichen Bestandteil umfasst; vorzugsweise wobei die Kartoffeltrockenflocken in einer Menge von 35 g bis 45 g, vorzugsweise 40 g, bezogen auf ein zuzusetzendes Volumen von 250 ml wässrigen Lösungsmittels vorliegen.

4. Zusammensetzung nach einem der Ansprüche 1 bis 3, wobei die Zusammensetzung in Form einer portionierten Einheit, vorzugsweise in einem Portionsbeutel oder in einer anderen Form der Verpackung portionierter Einheiten, wie Tüten, Sachets, Schläuche, Becher, Kartons vorliegt.

5. Zusammensetzung nach Anspruch 4, wobei die Verpackung mit einer Kennzeichnung versehen ist, die einen grafischen Code und/oder einen RFID- Tag umfasst.

6. Verwendung der Zusammensetzung nach einem der Ansprüche 1 oder 2 zur Herstellung einer Prüfanschmutzung zur Bewertung der Reinigungswirkung von Reinigungsvorrichtungen zur Reinigung von Urinflaschen für menschliche Ausscheidungen.

7. Verwendung der Zusammensetzung nach Anspruch 3 zur Herstellung einer Prüfanschmutzung zur Bewertung der Reinigungswirkung von Reinigungsvorrichtungen zur Reinigung von Steckbecken für menschliche Ausscheidungen.

8. Verwendung nach Anspruch 6 oder 7, wobei die Prüfanschmutzung durch Lösen der Zusammensetzung in einem wässrigen Medium hergestellt wird.

9. Verfahren zur Herstellung einer Prüfanschmutzung zur Bewertung der Reinigungswirkung von Reinigungsvorrichtungen zur Reinigung von Behältern für menschliche Ausscheidungen, insbesondere von Reinigungs-Desinfektionsgeräten, umfassend den Schritt des Lösens der Zusammensetzung nach einem der Ansprüche 1 bis 3 in einem wässrigen Medium; vorzugsweise wobei das wässrige Medium Wasser oder eine wässrige Pufferlösung aufweist.

10. Prüfanschmutzung zur Bewertung der Reinigungswirkung von Reinigungsvorrichtungen zur Reinigung von Behältern für menschliche Ausscheidungen, insbesondere von Reinigungs-Desinfektionsgeräten, die die Bestandteile der Zusammensetzung gemäß einem der Ansprüche 1 bis 3 in einem wässrigen Medium, vorzugsweise Wasser, umfasst.

11. Prüfanschmutzung zur Bewertung der Reinigungswirkung von Reinigungsvorrichtungen zur Reinigung von Behältern für menschliche Ausscheidungen, insbesondere von Reinigungs-Desinfektionsgeräten, die durch das Verfahren nach Anspruch 9 erhältlich ist.

12. Verwendung der Prüfanschmutzung nach Anspruch 10 oder 11 zur Bewertung der Reinigungswirkung von Reinigungsvorrichtungen zur Reinigung von Urinflaschen oder Steckbecken für menschliche Ausscheidungen.

13. Verwendung nach Anspruch 12, wobei die Prüfanschmutzung die Bestandteile der Zusammensetzung gemäß einem der Ansprüche 1 bis 3 in einem wässrigen Medium, vorzugsweise Wasser, umfasst und die Reinigungswirkung von Reinigungsvorrichtungen zur Reinigung von Urinflaschen für menschlichen Ausscheidungen bewertet wird.

14. Verwendung nach Anspruch 12, wobei die Prüfanschmutzung die Bestandteile der Zusammensetzung gemäß Anspruch 3 in einem wässrigen Medium, vorzugsweise Wasser, umfasst und die Reinigungswirkung von Reinigungsvorrichtungen zur Reinigung von Steckbecken für menschliche Ausscheidungen bewertet wird.

15. Verfahren zur Bewertung der Reinigungswirkung von Reinigungsvorrichtungen zur Reinigung von Behältern für menschliche Ausscheidungen, insbesondere von Reinigungs-Desinfektionsgeräten, umfassend:
(a) Herstellen einer Prüfanschmutzung mit einem Verfahren nach Anspruch 9;
(b) Auftragen der in Schritt (a) hergestellten Prüfanschmutzung an Behältern für menschliche Ausscheidungen, insbesondere Steckbecken oder Urinflaschen;
(c) Reinigen der Behälter aus Schritt (c) mittels einer zu bewertenden Reinigungsvorrichtung; und
(d) Bewertung der Reinigungswirkung der Reinigungsvorrichtung zur Reinigung von Behältern für menschliche Ausscheidungen anhand der Menge der vorhandenen oder entfernten Prüfanschmutzung.

## Claims

1. Composition comprising the following constituents in dry form: (a) nigrosin, (b) wheat flour and (c) whole egg or whole egg substitutes, such as vegetarian or vegan whole egg, or artificial compositions comprising the constituents of whole egg, wherein the constituents are present in the composition in the following amounts, based in each case on a volume of 250 ml of aqueous solvent to be added: (a) nigrosin 0.8 g to 1.2 g, preferably 1.0 g, (b) wheat flour 30 g to 40 g, preferably 35 g, and (c) whole egg 17 g to 21 g, preferably 19 g.

2. Composition according to Claim 1, wherein nigrosin and whole egg are in powder form.

3. Composition according to either of Claims 1 and 2, wherein the composition comprises potato dry flakes as an additional constituent; preferably wherein the potato dry flakes are present in an amount of 35 g to 45 g, preferably 40 g, based on a volume of 250 ml of aqueous solvent to be added.

4. Composition according to any of Claims 1 to 3, wherein the composition is present in form of a portioned unit, preferably a portion pack, or of units portioned in another form of packaging, such as bags, sachets, tubes, cups, cartons.

5. Composition according to Claim 4, wherein the package comprises a label comprising a graphic code and/or an RFID tag.

6. Use of the composition according to either of Claims 1 and 2 for production of test soil for assessment of the cleaning action of cleaning devices for cleaning of urine bottles for human egesta.

7. Use of the composition according to Claim 3 for production of test soil for assessment of the cleaning action of cleaning devices for cleaning of bedpans for human egesta.

8. Use according to Claim 6 or 7, wherein the test soil is produced by dissolving the composition in an aqueous medium.

9. Method for producing a test soil for assessment of the cleaning action of cleaning devices for cleaning of vessels for human egesta, especially of washer-disinfectors, comprising the step of dissolving the composition according to any of Claims 1 to 3 in an aqueous medium; preferably wherein the aqueous medium includes water or an aqueous buffer solution.

10. Test soil for assessing the cleaning action of cleaning devices for cleaning of vessels for human egesta, especially of washer-disinfectors, comprising the constituents of the composition according to any of Claims 1 to 3 in an aqueous medium, preferably water.

11. Test soil for assessing the cleaning action of cleaning devices for cleaning of vessels for human egesta, especially of washer-disinfectors, which is obtainable by the method according to Claim 9.

12. Use of the test soil according to Claim 10 or 11 for assessment of the cleaning action of cleaning devices for cleaning of urine bottles or bedpans for human egesta.

13. Use according to Claim 12, wherein the test soil comprises the constituents of the composition according to any of Claims 1 to 3 in an aqueous medium, preferably water, and the cleaning action of cleaning devices for cleaning of urine bottles for human egesta is assessed.

14. Use according to Claim 12, wherein the test soil comprises the constituents of the composition according to Claim 3 in an aqueous medium, preferably water, and the cleaning action of cleaning devices for cleaning of bedpans for human egesta is assessed.

15. Method of assessing the cleaning action of cleaning devices for cleaning of vessels for human egesta, especially of washer-disinfectors, comprising:
(a) producing test soil by a method according to Claim 9;
(b) applying the test soil produced in step (a) to vessels for human egesta, especially bedpans or urine bottles;
(c) cleaning the vessels from step (c) by means of a cleaning device to be assessed; and
(d) assessing the cleaning action of the cleaning device for cleaning vessels for human egesta by the amount of test soil present or removed.

## Revendications

1. Composition, qui comprend les composants suivants sous une forme sèche : (a) de la nigrosine, (b) de la farine de blé et (c) de l'œuf entier ou des substituts d'œuf entier, comme de l'œuf entier végétarien ou végan ou des compositions artificielles qui comprennent les composants de l'œuf entier, dans laquelle les composants sont présents en les quantités suivantes, respectivement par rapport à un volume à ajouter de 250 ml de solvant aqueux dans la composition : (a) nigrosine 0,8 g à 1,2 g, de préférence 1,0 g, (b) farine de blé 30 g à 40 g, de préférence 35 g et (c) œuf entier 17 g à 21 g, de préférence 19 g.

2. Composition selon la revendication 1, dans laquelle la nigrosine et l'œuf entier se présentent sous forme de poudre.

3. Composition selon l'une des revendications 1 ou 2, où la composition comprend des flocons déshydratés de pommes de terre en tant que composant additionnel ; de préférence dans laquelle les flocons déshydratés de pommes de terre sont présents en une quantité de 35 g à 45 g, de préférence 40 g, par rapport à un volume à ajouter de 250 ml de solvant aqueux.

4. Composition selon l'une des revendications 1 à 3, où la composition se présente sous la forme d'une unité portionnée, de préférence dans un sachet-dose ou sous une autre forme d'emballage d'unités portionnées, telle que des sacs, des sachets, des tubes, des pots, des cartons.

5. Composition selon la revendication 4, dans laquelle l'emballage est pourvu d'une caractérisation qui comprend un code graphique et/ou une étiquette RFID.

6. Utilisation de la composition selon l'une des revendications 1 ou 2 pour la production d'une salissure test pour l'évaluation de l'efficacité de nettoyage des dispositifs de nettoyage destinés au nettoyage des flacons d'urine pour les excrétions humaines.

7. Utilisation de la composition selon la revendication 3 pour la production d'une salissure test pour l'évaluation de l'efficacité de nettoyage des dispositifs de nettoyage destinés au nettoyage des bassins hygiéniques pour les excrétions humaines.

8. Utilisation selon la revendication 6 ou 7, dans laquelle la salissure test est produite par dissolution de la composition dans un milieu aqueux.

9. Procédé de production d'une salissure test pour l'évaluation de l'efficacité de nettoyage des dispositifs de nettoyage destinés au nettoyage de contenants pour les excrétions humaines, en particulier des appareils de nettoyage-désinfection, comprenant l'étape de dissolution de la composition selon l'une des revendications 1 à 3 dans un milieu aqueux ; de préférence dans lequel le milieu aqueux contient de l'eau ou une solution aqueuse tampon.

10. Salissure test pour l'évaluation de l'efficacité de nettoyage des dispositifs de nettoyage destinés au nettoyage de contenants pour les excrétions humaines, en particulier des appareils de nettoyage-désinfection, qui comprend les composants de la composition selon l'une des revendications 1 à 3 dans un milieu aqueux, de préférence de l'eau.

11. Salissure test pour l'évaluation de l'efficacité de nettoyage des dispositifs de nettoyage destinés au nettoyage de contenants pour les excrétions humaines, en particulier des appareils de nettoyage-désinfection, qui peut être obtenue par le procédé selon la revendication 9.

12. Utilisation de la salissure test selon la revendication 10 ou 11 pour l'évaluation de l'efficacité de nettoyage des dispositifs de nettoyage destinés au nettoyage des flacons d'urine ou des bassins hygiéniques pour les excrétions humaines.

13. Utilisation selon la revendication 12, dans laquelle la salissure test comprend les composants de la composition selon l'une des revendications 1 à 3 dans un milieu aqueux, de préférence de l'eau, et l'efficacité de nettoyage des dispositifs de nettoyage destinés au nettoyage des flacons d'urine pour les excrétions humaines est évaluée.

14. Utilisation selon la revendication 12, dans laquelle la salissure test comprend les composants de la composition selon la revendication 3 dans un milieu aqueux, de préférence de l'eau, et l'efficacité de nettoyage des dispositifs de nettoyage destinés au nettoyage des bassins hygiéniques pour les excrétions humaines est évaluée.

15. Procédé d'évaluation de l'efficacité de nettoyage des dispositifs de nettoyage destinés au nettoyage de contenants pour les excrétions humaines, en particulier d'appareils de nettoyage-désinfection, comprenant :
(a) la production d'une salissure test avec un procédé selon la revendication 9 ;
(b) l'application de la salissure test produite à l'étape (a) sur des contenants pour les excrétions humaines, en particulier les bassins hygiéniques ou les flacons d'urine ;
(c) le nettoyage des contenants de l'étape (c) au moyen d'un dispositif de nettoyage à évaluer ; et
(d) l'évaluation de l'efficacité de nettoyage des dispositifs de nettoyage destinés au nettoyage de contenants pour les excrétions humaines à l'aide de la quantité de salissures tests présentes ou éliminées.
